# EUROPEAN PATENT APPLICATION

(11) **EP 4 353 356 A1**
(43) Date of publication of application: **17.04.2024**
(21) Application number: 22201660.2
(22) Date of filing: 14.10.2022
(51) Int. Cl.: B01J 23/72, B01J 37/03, B01J 35/02, C07C 43/00

(54) **BIFUNCTIONAL CATALYST FORMULATION FOR SYNTHESIS OF DIMETHYLETHER (DME) FROM WASTE CARBON DIOXIDE AND HYDROGEN BY ONE-STEP AND THE SYNTHESIS METHOD THEREOF**

(71) Applicant: Socar Turkey Arastirma Gelistirme Ve Inovasyon A.S., 35800 Aliaga/Izmir (TR); Izmir Yuksek Teknoloji Enstitusu Rektorlugu, Izmir (TR)
(72) Inventor: SEKER, Erol, Izmir (TR); ZAHIDOVA, Aysel, Izmir (TR); MUTLU, Vahide Nuran, Izmir (TR); DELIISMAIL, Ozgun, Izmir (TR); SARRAFI, Sahin, Izmir (TR); TUNCER, Basak, Izmir (TR)
(74) Representative: Sevinç, Cenk

(57) **Abstract**

This invention relates to bifunctional catalyst formulation for synthesis of dimethyl-ether (DME) from waste carbon dioxide (CO₂) and hydrogen (H₂) and the preparation method of this catalyst formulation. In the preparation method, aluminum oxide-silicon dioxide-copper oxide (Al₂O₃-SiO₂-CuO) catalyst mixture formulation is obtained by using the sol-gel method. The catalysts that are the subject of the invention are bifunctional and perform the synthesis of DME from CO₂ and H₂ in a single-step without forming unwanted byproduct of CH₄, and thus, the synthesis of DME can be done much simply and economically. In addition to this, the sol-gel method in the current invention yields the controlled crystallite size of the active component in the catalysts; thus, the active site loss problem due to uncontrolled growth of crystal sizes that occurs in traditional preparation methods is prevented. The catalyst mixture formulation weight percentages are 10%-90% Al2O₃, 10%-90% SiO₂ and 10%-90% CuO.

## Description

### Related Technical Field

Present invention relates to bifunctional catalyst formulation for synthesis of dimethyl-ether (DME) from waste carbon dioxide (CO₂) and hydrogen (H₂) in one-step and synthesis method of this catalyst formulation. In this method, aluminum oxide-silicon dioxide-copper oxide (Al₂O₃-SiO₂-CuO) catalyst mixture formulation is obtained by using sol-gel method.

### State of the art

Dimethyl ether (DME) is an intriguing molecule that can have impact ranging from being ultra low carbon to carbon negative on the environment. It can significantly reduce carbon footprint of transportation sector beyond as an energy-dense, cost-effective means to move renewable hydrogen, as a blending agent for propane, and as a diesel replacement. DME has been used for decades as an environmentally safe, non-toxic aerosol propellant as well as to blend with propane. It can be produced domestically from a variety of feedstocks, including dairy biogas, food waste, and waste streams from industrial processes.#DME offers a 68-101% reduction in Greenhouse Gases[1]. An article titled as "Dimethyl Ether as the Next Generation Fuel to Control Nitrogen Oxides and Particulate Matter Emissions from Internal Combustion Engines" which is published by Yanuandri Putrasari and Yanuandri Putrasari mentions that dimethyl ether is a promising fuel[2]. As a result, considering the wide range of uses of dimethyl ether, studies on the synthesis of dimethyl ether have been increasing for decades.

In the prior art, there are studies on the production of dimethyl ether using a mixture of carbon monoxide, carbon dioxide and hydrogen on solid catalysts prepared using different methods and catalyst formulations/combinations. Generally, two-step methods using different catalyst formulations and feed mixtures are used in these studies. In the first step, methanol is produced from syngas with a commercial methanol synthesis catalyst or a similar formulation, and in second step, DME is produced by dewatering the methanol on acid solid catalysts (zeolite, etc.). Unfortunately, this approach has disadvantages such as requiring high energy intensity and the usage of syngas. Syngas, also known as synthesis gas, is often produced from a catalyzed reaction of a mixture of steam and methane (steam methane reforming). Syngas contains carbon monoxide (CO), which is a chemical asphyxiant. Toxic action of carbon monoxide is caused by combining with the hemoglobin in the blood to form the relatively stable carboxyhemoglobin. The stability of the carboxyhemoglobin prevents oxygen from being taken into the body and consequently the body is deprived of needed oxygen[3].

In the state of the art, also some common techniques exists for DME synthesis from carbon dioxide and hydrogen or from carbon monoxide and carbon dioxide with two-step approach. In first step, methanol is obtained from a mixture of CO/CO₂ and H₂, then DME synthesis is carried out by dehydration of methanol. In this approach, separate catalysts and reactors are needed in both steps and purification processes are also needed. This situation brings along a more complex synthesis process as well as high investment and operating costs.

In the prior art, there are studies on catalysts and processes that enable DME synthesis from carbon dioxide, carbon monoxide and hydrogen with a one-step direct approach. In this approach copper oxide/zinc oxide (CuO/ZnO) based methanol synthesis catalyst composition is used. It should also be noted that methanol synthesis catalyst with CuO/ZnO based composition is also used in two-step DME synthesis. The main problem of the methanol synthesis catalyst is that it is pyrophoric. Pyrophoric materials are substances that ignite spontaneously upon exposure to oxygen or air. They can also be water-reactive, where heat and hydrogen (a flammable gas) are produced. Other common hazards include corrosivity, teratogenicity, and organic peroxide formation, along with damage to the liver, kidneys, and central nervous system. Failure to follow proper handling techniques during the use of any pyrophoric chemical (like as methanol synthesis catalyst) may result in serious injury or death. In addition to this, methanol synthesis catalyst is difficult to store and transport. Also, the methanol synthesis catalyst is deactivated due to sintering process and undesired contaminants. In addition, in the synthesis of DME in one-step, the methanol synthesis catalyst needs to be mixed with other oxides with redox properties. As a result of mixing the methanol synthesis catalyst with other oxides, there is a loss of activity in the methanol synthesis catalyst and the acidic properties of the methanol synthesis catalyst change undesirably.

In the state of the art, in a thesis published by Mehmet Ali Sener, a bifunctional catalyst formulation was developed for one-step DME synthesis using syn-gas and CO₂ containing syn-gas. The mentioned catalyst formulation consists of a mesoporous carbon (CMK-3) support material loaded with TPA (tungstophosphoric acid) and copper-zinc (Cu-Zn) metals. Mesoporous carbon and tungstophosphoric acid are high cost materials [4]. Also, syn-gas and CO₂ containing syn-gas mixtures were the input reactants, instead of just using only CO₂/H₂ mixture as the reactants. In another similar prior art, in a Tubitak project carried out by Nuray Oktar, studies were carried out on the synthesis of dimethyl ether methanol from syngas rich in carbon dioxide with bifunctional catalyst systems. In this study, two different copper-zinc oxide-aluminum oxide (Cu-ZnO-Al₂O₃) and copper-zinc oxide-zirconium dioxide (Cu-ZnO-ZrO₂) catalysts are used as methanol synthesis catalysts, and costly tungstophosphoric acid (TPA) is used to increase the activity of these catalysts [5].

In the prior art, there are many problems: (1) usage of syngas which contains toxic carbon monoxide (CO) during the two-step DME synthesis, (2) high energy density requirement for the two-step DME synthesis, (3) syngas is produced from methane steam reforming, thus resulting in unwanted carbon footproint, (4) requirement of separate catalysts and reactors for the two-step DME synthesis, which also bring along more complex synthesis process as well as high investment and operating costs, (5) pyrophoric catalyst (CuO/ZnO) which ignites instantly upon exposure to oxygen requirement for the one-step DME synthesis. Because of these problems in prior art, developments, related to usage of waste CO₂ instead of syngas, in this technical field are required.

### Advantages Provided by the Invention

In the present invention, bifunctional catalyst formulation for synthesis of dimethyl-ether (DME) from just using waste carbon dioxide (CO₂) and hydrogen (H₂) in one-step without forming unwanted byproduct of CH₄ and also, the preparation synthesis of this catalyst formulation is disclosed. In the preparation method, aluminum oxide-silicon dioxide-copper oxide (Al₂O₃-SiO₂-CuO) catalyst mixture formulation is obtained by using a sol-gel method. The catalysts that are the subject of the invention are bifunctional and perform the synthesis of DME from CO₂ and H₂ in a single-step, and thus, the synthesis of DME can be done much simply and economically. In addition to this, the usage of the sol-gel method of the current invention ensures the control of the crystallite size of the active component even in its high loadings (e.g. >50 wt%) in the catalysts; thus, the active sites loss problem that typically occurs in case of uncontrolled growth of crystallite sizes observed on catalysts synthesized using traditional preparation methods, such as impregnation or co-precipitation, is prevented.

The most important object of the present invention is to enable a bifuctional catalyst formulation which prevents requirement of complex processes and high CAPEX/OPEX costs in two-steps DME synthesis. In other words, the feature of the catalyst prevents high CAPEX/OPEX costs and complex process requirements associated with two-step DME synthesis. In the present invention, DME synthesis is carried out in a single-step over catalysts containing copper oxide as an active component in the range of 10-90wt% in the Al₂O₃-SiO₂ mixture prepared with the sol-gel method. Such high loadings (e.g. 10-90wt% CuO) of active components with the controlled crytallite size in a catalyst cannot be achieved using traditional preparation methods, such as impregntaion, co-precipitation, ion-exchange etc. In fact, bifunctional catalyst required for a single-step DME synthesis from CO₂ and H₂ must have the catalytic properties having both reduction/oxidation and acidic functions. In the combination of Al₂O₃-SiO₂-CuO, the property required for methanol synthesis comes from the CuO and Al₂O₃ components, and the acidic property required for methanol dehydration comes from the Al₂O₃-SiO₂ component. Through the synthesis method of this formulation, these two catalytic functions meet in a single catalyst combination. In other words, In the indirect method, there are two reactions, methanol synthesis from the syngas ( a mixture of CO and H₂) and Methanol dehydration reaction (DME synthesis). For these two reactions, two different catalysts and two reactors are required. However, in one-step method, which is the concern of the prsent invention, there is only one catalyst and one reactor to convert CO₂ and H₂ (instead of syngas mixture) to DME.

Another object of present invention is to synthesize a single catalyst with two properties which are reduction/oxidation and acidity. A single catalyst with two functions can be obtained using the method of the invention. In the combination of Al₂O₃-SiO₂-CuO in the catalyst, the catalytic feature required for methanol synthesis comes from mainly CuO and also Al₂O₃ components, and the acidic feature required for methanol dehydration comes from the Al₂O₃-SiO₂ component.

Another object of the invention is to ensure that the catalyst crystallite size used in the one-step DME synthesis is at the desired (stable) dimensions. The crystallite size of catalysts plays a prominent role in defining the performance, the selectivity of products, and the life span of the catalyst. In addition, thanks to the Al₂O₃-SiO₂-CuO catalyst with triple oxide compound formulation synthesized by using the sol-gel method of the invention, which is specific to the catalyst formulation of the invention, the properties of the catalyst such as surface area, pore and crystal size and crystal structure can be adjusted. In other words, the problem of active sites loss, which occurs in case of uncontrolled growth of crystallite sizes, is prevented. More specifically, in the present invention, addition of copper nitrate to the solution during the sol-gel method ensures that the desired crystallite size of the active component in catalysts with high copper oxide loadings is at the desired (stable) dimensions. These physico-chemical properties (crystallite size, surface area and etc.) of the catalysts are highly effective on the DME synthesis reaction activity and selectivity in one-step.

Another object of the present invention is to provide a safe method for obtaining DME from waste carbon dioxide (CO₂) and hydrogen (H₂). The mentioned safe method is provided by using a catalyst that does not show pyrophoric properties in the present invention, unlike the copper oxide/zinc oxide (CuO/ZnO) catalyst (methanol synthesis catalyst) used for the DME synthesis in the state of the prior art. Failure to follow proper handling techniques during the use of any pyrophoric chemicals (such as methanol synthesis catalyst (CuO/ZnO)) may result in serious injury or death. Pyrophoric materials are substances that could ignite instantly upon exposure to oxygen or air. Unlike DME synthesis catalysts, which are in the state of the art and show pyrophoric properties, the bifunctional catalyst formulation used in DME synthesis, which is the subject of the invention, does not show pyrophroic properties.

Present invention overcomes the problems present in the prior art by preventing requirement of complex processes and high CAPEX/OPEX costs in two-step DME synthesis, offering a single catalyst with two functions, providing a safe method for obtaining DME from waste carbon dioxide (CO₂) and hydrogen (H₂) without forming unwanted byproduct of CH₄ and enabling a desired crystallite size for the bifunctional catalyst. With the help of the method according to the invention, even when the amount of active component CuO is 90%, the crystallite sizes remain constant in the range of 30-50nm. This sol-gel method prevents the active component from sintering. In other words, the dimensions of the catalytic reactors, designed using the catalysts of the current invention, will not increase; thus, the DME synthesis processes will be economical. Also, unlike the prior art, only one catalyst is enough to carry out one-step DME synthesis. This also lowers the DME production cost.

### Brief Description of the Drawings

**Figure 1****.** X-Ray diffraction (XRD) analysis results of 30% Al₂O₃-70% SiO₂-(0-90%) CuO catalysts calcinated for 3 hours at different calcination temperatures (400°C, 500°C, 600°C)
**Figure 2****.** X-Ray diffraction (XRD) analysis results of 30% Al₂O₃-70% SiO₂-(0-90%) CuO catalysts calcinated for 6 hours at different calcination temperatures (400°C, 500°C, 600°C)
**Figure 3****.** X-Ray diffraction (XRD) analysis results of 60% Al₂O₃-40% SiO₂-(0-90%) CuO catalysts calcinated for 3 hours at different calcination temperatures (400°C, 500°C, 600°C)
**Figure 4****.** X-Ray diffraction (XRD) analysis results of 60% Al₂O₃-40% SiO₂-(0-90%) CuO catalysts calcinated for 6 hours at different calcination temperatures (400°C, 500°C, 600°C)
**Figure 5****.** X-Ray diffraction (XRD) analysis results of 80% Al₂O₃-20% SiO₂-(0-90%) CuO catalysts calcinated for 3 hours at different calcination temperatures (400°C, 500°C, 600°C)
**Figure 6****.** X-Ray diffraction (XRD) analysis results of 80% Al₂O₃-20% SiO₂-(0-90%) CuO catalysts calcinated for 6 hours at different calcination temperatures (400°C, 500°C, 600°C)
**Figure 7****.** Brunauer-Emmett-Teller (BET) isotherms of 30% Al₂O₃-70% SiO₂-(0-90%) CuO catalysts synthesized at different rates and different calcination temperatures
**Figure 8****.** Brunauer-Emmett-Teller (BET) isotherms of 60% Al₂O₃-40% SiO₂-(0-90%) CuO catalysts synthesized at different rates and different calcination temperatures
**Figure 9****.** Brunauer-Emmett-Teller (BET) isotherms of 80% Al₂O₃-20% SiO₂-(0-90%) CuO catalysts synthesized at different rates and different calcination temperatures
**Figure 10****.** Methanol conversion results in methanol dehydration reaction over 30% Al₂O₃-70% SiO₂-(0-90%) CuO catalysts at different temperatures (200°C -350°C)
**Figure 11****.** Methanol conversion results in methanol dehydration reaction over 60% Al₂O₃-40% SiO₂-(0-90%) CuO catalysts at different temperatures (200°C -350°C)
**Figure 12****.** Methanol conversion results in methanol dehydration reaction over 80% Al₂O₃-20% SiO₂-(0-90%) CuO catalysts at different temperatures (200°C -350°C)
**Figure 13****.** DME selectivity results among the products formed as a result of the reaction over 30% Al₂O₃-70% SiO₂-(0-90%) CuO catalysts at different temperatures (200°C -350°C)
**Figure 14****.** DME selectivity results among the products formed as a result of the reaction over 60% Al₂O₃-40% SiO₂-(0-90%) CuO catalysts at different temperatures (200°C -350°C)
**Figure 15****.** DME selectivity results among the products formed as a result of the reaction over 80% Al₂O₃-20% SiO₂-(0-90%) CuO catalysts at different temperatures (200°C -350°C)

### Description of the Invention

This invention relates to bifunctional catalyst formulation for synthesis of dimethyl-ether (DME) from waste carbon dioxide (CO₂) and hydrogen (H₂) in one-step without forming unwanted byproduct of CH₄ and synthesis method of this catalyst formulation. In the synthesis method, aluminum oxide-silicon dioxide-copper oxide (Al₂O₃-SiO₂-CuO) catalyst mixture formulation is prepared by using sol-gel method. The catalysts that are the subject of the invention are bifunctional and perform the synthesis of DME from CO₂ and H₂ in a single-step without forming unwanted CH₄, and thus, the synthesis of DME can be done much simply and economically. In addition to this, crystallite size of the active component in the catalysts using the sol-gel method of the current invention is ensured in desired (stable) dimensions, and thus, the active sites loss problem that occurs in case of uncontrolled growth of crystallite sizes is prevented. The catalyst mixture formulation weight percentages are 10%-90% Al₂O₃, 10%-90% SiO₂ and 10%-90% CuO.

Bifunctional catalyst formulation for synthesis of dimethyl-ether (DME) from waste carbon dioxide (CO₂) and hydrogen (H₂) and one-step catalyst preparation method comprises the following steps:
i. mixing of tetraethyl orthosilicate (TEOS) precursor which is used as silicon (Si) source, ethanol as solvent, deionized water for hydrolysis and 1 M hydrochloric acid (HCI) at 50-85°C for 1-5 hours and obtaining SiO₂ sol without precipitation,
ii. adding aluminum isopropoxide (AIP) precursor used as an aluminum (Al) source into deionized water at 75-95°C and mixing for 0.5-3 hours,
iii. adding 65% nitric acid (HNO₃) to the resulting mixture and mixing for 0.5-3 hours and obtaining Al₂O₃ sol without precipitation,
iv. adding the SiO₂ sol prepared in the process step (i) into the Al₂O₃ sol and applying the mixing process for 15-45 minutes, to obtain the desired weight percentages of 10%-90% Al₂O₃ and 90%-10% SiO₂,
v. adding an aqueous solution of copper nitrate ([Cu(NO₃)₂.3H₂O]) to the resulting mixture and applying the mixing process at 75-95°C for 0.5-3 hours,
vi. slow evaporation of the resulting mixture at a temperature of 50-70°C and gelling the mixture for 3-6 hours,
vii. drying the obtained gel in static air or air flow for 1-24 hours at 90-150°C,
viii. reducing the dried catalyst to 100 mesh - 270 mesh size by grinding and then calcining at 300-700°C for 3-9 hours at different temperatures and times.

By the way of reducing the dried catalyst to 100 mesh-270 mesh size by grinding, mass transfers are made easier and thus the stability of the crystal size in the catalysts to be obtained is maintained at certain temperatures.

A bifunctional catalyst having both reduction/oxidation and acidic characteristics is necessary for the single-step production of DME from CO₂ and H₂. The CuO component of Al₂O₃-SiO₂-CuO in contact with the interaction with Al₂O₃ contains the property necessary for methanol production, while Al₂O₃-SiO₂ contains the acidic property necessary for methanol dehydration. These two catalytic functionalities come together in a single catalyst combination achieved using the preparation procedure in the current invention.

Using the method of the invention, a single catalyst can serve two purposes. The catalytic property needed for methanol synthesis comes from the combination of Al₂O₃-SiO₂-CuO, while the acidic property needed for methanol dehydration comes from the Al₂O₃-SiO₂ component.

The performance, product selectivity, and lifetime of catalysts are significantly influenced by the crystallite size of the catalysts. Additionally, the Al₂O₃-SiO₂-CuO catalyst with triple oxide formulation, unique to the catalyst formulation of the invention, was created using the sol-gel method. This allowed the catalyst's attributes, such as surface area, pore size, and crystal structure, to be changed. In other words, the issue of active site loss, which happens when crystallite sizes grow without control, is avoided. More specifically, in the present invention, copper nitrate is added to the solution during the sol-gel synthesis to guarantee that the active component's crystallite size is at the appropriate (stable) dimensions in catalysts even with high copper oxide loadings. Crystallite size, surface area, and other physico-chemical characteristics of the catalysts have a significant impact on the DME synthesis reaction activity and selectivity in a single-step from CO₂ and H₂.

In contrast to the copper oxide/zinc oxide (CuO/ZnO) catalyst (methanol synthesis catalyst) employed for the DME synthesis, the state of the art of current invention is to eliminate pyrophoric properties that could cause serious harm or even death. Materials classified as pyrophoric could ignite instantly when exposed to oxygen or air. The bifunctional catalyst formulation employed in DME synthesis, which is the subject of the invention, does not exhibit pyrophoric properties, in contrast to traditional DME synthesis catalysts.

By eliminating the need for complicated procedures and additional costs in one-step DME synthesis, providing a single catalyst with two functions, offering a secure method for producing DME from waste carbon dioxide (CO₂) and hydrogen (H₂), and enabling a desired crystallite size in the bifunctional catalyst; thus, the present invention solves the issues present in the prior art. With the aid of the approach of the invention, the crystallite sizes stay constant in 30-50nm range even when the active component CuO content reaches 90wt%. As a result, the active component doesn't sinter which lead to cost-effective catalytic reactors. Additionally, unlike the prior inventions, a one-step DME synthesis using current invention can be achieved using just one catalyst. By this way, CAPEX and OPEX costs of DME production can be reduced.

As seen in the step i and ii, SiO₂ sol and Al₂O₃ sol are prepared separately. Because, the hydrolysis rates of Al and Si starting materials (i.e. Aluminum Isopropoxide and TetraEthyl OrthoSilicate (TEOS)) are very different and also, both starting materials require different sol-gel procedures. Thus, by completing the hydrolysis in a separate environment, one of Al₂O₃ or SiO₂ phases is prevented from being dominant during gelation. By preparing sol in separate environments and mixing, gelling is achieved and the formation of a homogeneous Al₂O₃ and SiO₂ mixture is ensured.

As seen in step i, in order to ensure that SiO₂ sol is homogeneous and stable, the precursor (TEOS) selected to prepare the SiO₂ sol, temperature and mixing time to prepare the SiO₂ sol, as well as the added amounts of ethanol, HCI and water, have been calculated and tested specific to desired catalyst formulation.

As seen in step ii and iii, in order to ensure that Al₂O₃ sol to be homogeneous and stable, the precursor (AIP) selected to prepare the Al₂O₃ sol, the temperature and mixing time, as well as the added water and 65% HNO₃, calculated and tested specific to desired catalyst formulation.

Although the step of adding HNO₃ (in step iii) is generally used in sol-gel methods, the amount of HNO₃ added, reaction temperature and mixing time are optimized specifically for the catalyst formulation of the invention; thus, being unique to formulation of catalyst in the current invention. Nitric acid was used as catalyst, can influence both the hydrolysis and condensation rates and the structure of the catalysts.

Since the SiO₂ and Al₂O₃ sols obtained as a result of the sol-gel steps i and iii are obtained without any precipitation, the dominance of any of the Al₂O₃ or SiO₂ phases during gelation is prevented. Therefore, as a result of the sol-gel step iv, a mixture of superior stability is obtained.

In the sol-gel step v, the addition of copper nitrate to the sols during the synthesis in the sol-gel method ensures that the crystallite size of the active component, CuO, in catalysts with high copper oxide loadings is at the desired (stable) dimensions. In other words, the properties of the catalyst such as surface area, pore and crystallite size and crystal structure can be adjusted. In that way, the problem of active site loss, which occurs in case of uncontrolled growth of crystallite sizes, is prevented.

Although the drying process of the catalysts mentioned in the process step vii in the invention is a process step used in all catalyst preparation methods, the drying temperature, environment and times affect the crystal structures and/or crystallite sizes that will affect the calcination and/or reduction process steps to be applied later. For this reason, the process step vii is specific to the preparation of the SiO₂-Al₂O₃ supported CuO catalysts with CuO high loadings, which is the subject of the invention.

As can be seen from the sol-gel step viii, the catalyst is subjected to high temperatures in the calcination step. This is to remove impurities coming from the starting materials. Although the calcination of the catalysts mentioned in the sol-gel step viii of the invention is a step used in all the catalyst preparation methods, calcination conditions vary depending on the catalyst formulations and reactions that are carried out. For example, in the method of the invention, the dried catalysts are ground in sizes of 100 mesh - 270 mesh and subjected to calcination at temperatures of 300-700°C for 3-9 hours. By using tailored calcination parameters for the catalysts mentioned in the method of invention, mass transfers in calcination are made easier and thus the stability of the crystallite size in the catalysts to be obtained is maintained at certain temperatures.

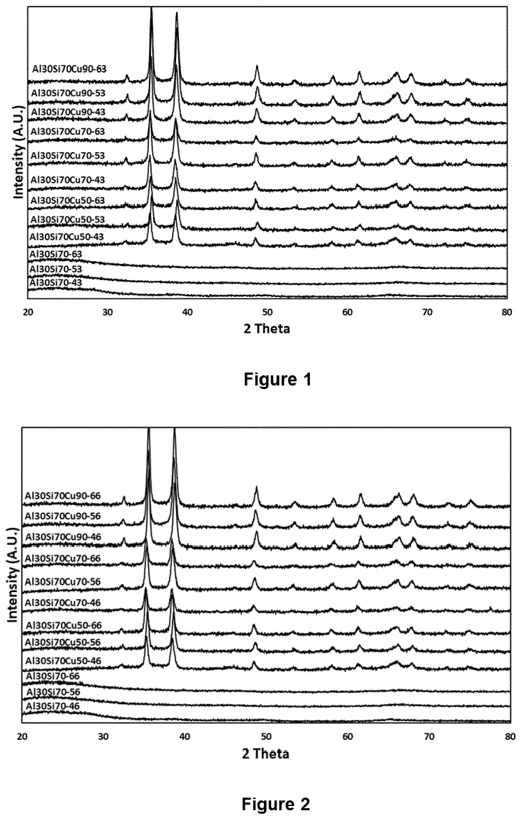

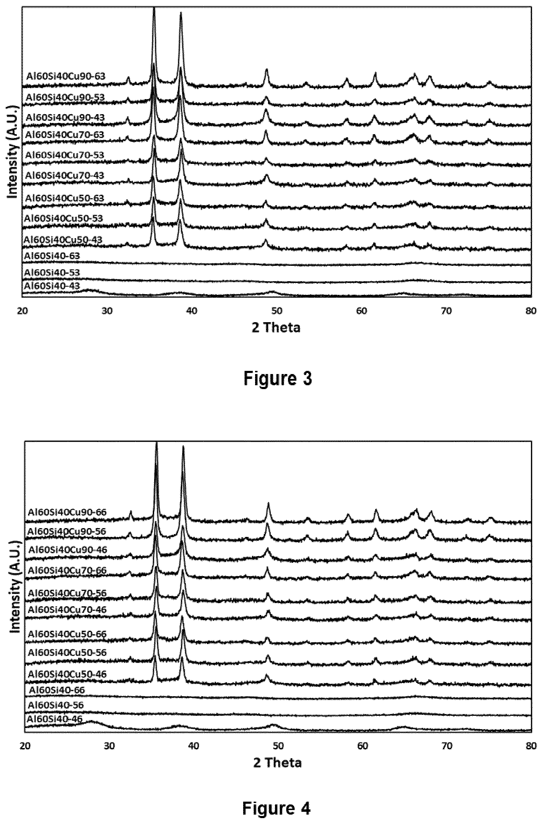

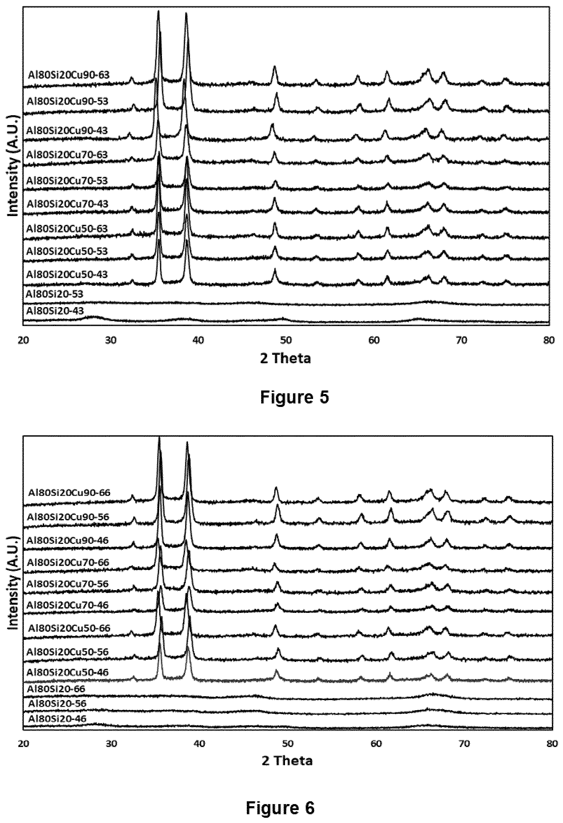

Figure 1-6 shows the XRD analysis results of Al₂O₃-SiO₂-CuO catalysts synthesized by the method according to the invention at different CuO loadings and different calcination temperatures and times for DME synthesis. With the help of the method according to the invention, even when the amount of active component CuO is 90wt%, the crystallite sizes remain constant in the range of 30-50nm. This prevents the active component from sintering. In other words, the dimensions of the catalytic reactors designed using the catalyst formulation prepared with the method of the invention will not increase.

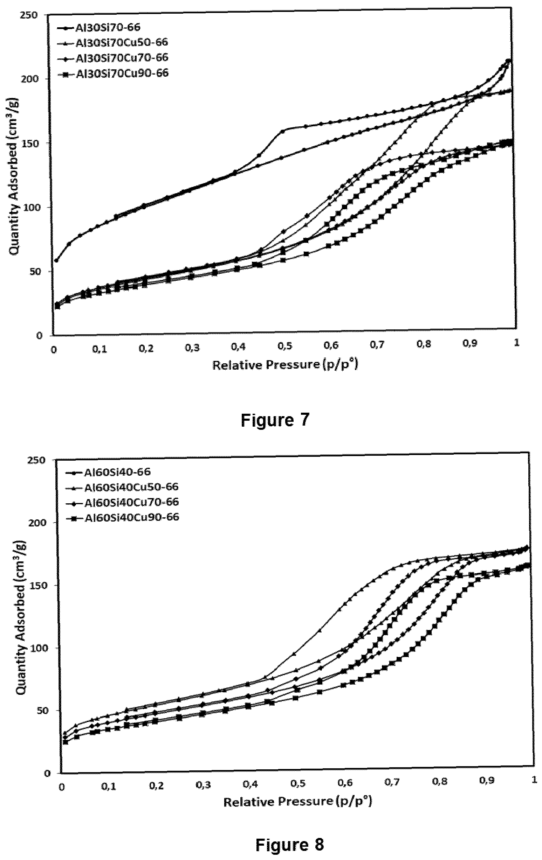

Figure 7-9 shows the Brunauer-Emmett-Teller (BET) isotherms of Al₂O₃-SiO₂-CuO catalysts synthesized with different CuO loadings and different calcination temperatures. All the catalysts without CuO contain slit shaped mesopores, having pore diameters of ∼4 nm, and high BET surface areas while the addition of CuO results in decrease of BET surface area and also the pore geometry changes to bottle-neck type pores having pore diameter of 5-6nm; as seen in Table 1. The sol-gel method of the current invention result in high BET surface area and mesopores centered around 5 nm even though the high calcination temperature and duration were used. In other words, the higher the surface area with high CuO loadings (e.g. 90%), the lower the amount of catalyst necessary to achieve desired product amounts. The use of the catalysts made using the formulation and sol-gel method of the invention ultimately results in small reactor sizes; hence, lower CAPEX and OPEX.

**Table 1. Surface area, surface volume and pore diameters of Al₂O₃-SiO₂-CuO catalysts synthesized with different CuO loadings and different calcination temperatures.**

| | S_{BET} (m²/g) | Vpore_{BJH} (cm³/g) | dporeBJH (A) |
|---|---|---|---|
| Al30Si70-66 | 345.73 | 0.27 | 41.05 |
| Al30Si70Cu50-66 | 152.35 | 0.29 | 61.55 |
| Al30Si70Cu70-66 | 153.71 | 0.23 | 50.39 |
| Al30Si70Cu90-66 | 135.46 | 0.22 | 59.35 |
| Al60Si40-66 | 366.30 | 0.40 | 38.1 |
| Al60Si40Cu50-66 | 187.66 | 0.28 | 50.09 |
| Al60Si40Cu70-66 | 161.92 | 0.27 | 60.82 |
| Al60Si40Cu90-66 | 139.58 | 0.24 | 64.61 |
| Al80Si20-66 | 274.28 | 0.36 | 41.72 |
| Al80Si20Cu50-66 | 149.97 | 0.23 | 46.98 |
| Al80Si20Cu70-66 | 132.62 | 0.20 | 46.50 |
| Al80Si20Cu90-66 | 115.59 | 0.13 | 42.72 |

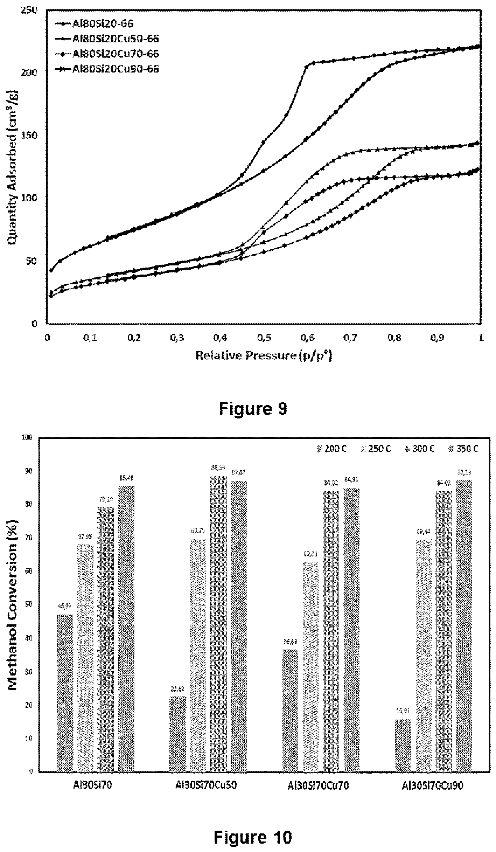

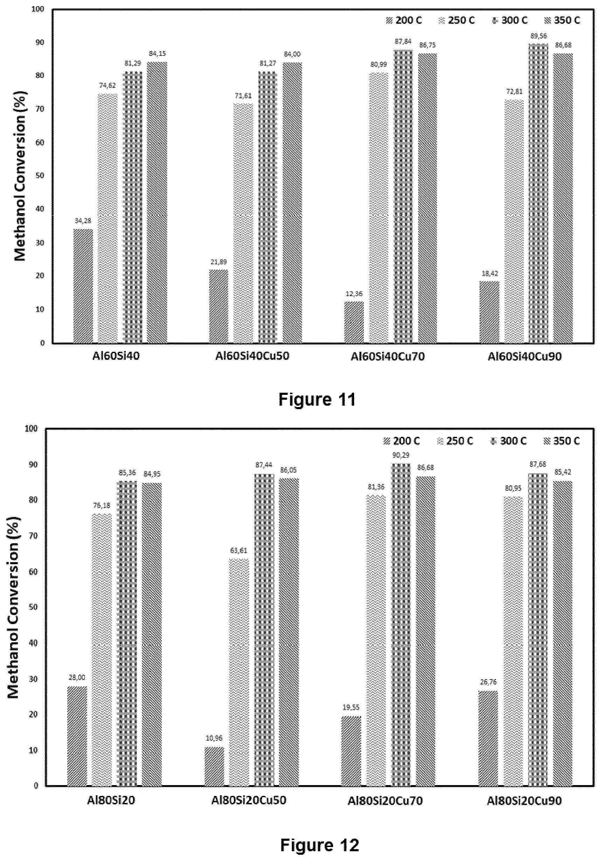

Figure 10-12 shows the methanol conversion results in methanol dehydration reaction in order to determine the activities of the synthesized catalysts, the conversion of methanol and the selectivity to DME formed as a result of the methanol dehydration reaction were calculated and It has been observed that the methanol conversion is low at low temperatures, reaching up to 87% at high temperatures.

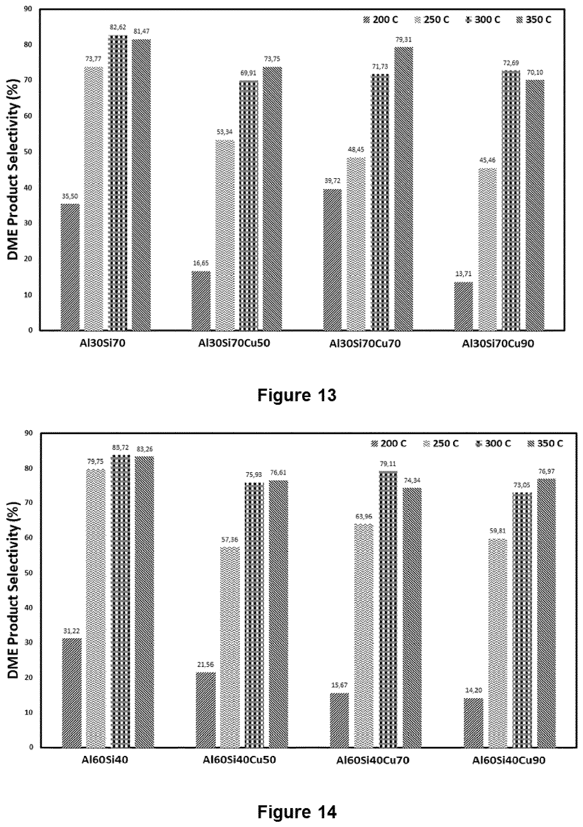

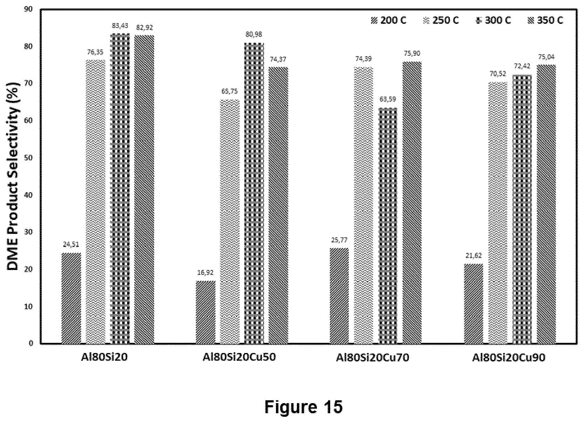

Figure 13-15 shows the DME selectivity results among the products formed as a result of the reaction. Among the products formed as a result of the reaction, the selectivity of DME was calculated and the highest DME selectivity was found to be 83%.

Another embodiment of the invention; bifunctional catalyst formulation for synthesis of dimethyl-ether (DME) from waste carbon dioxide (CO₂) and hydrogen (H₂) and single step preparation method comprises the following steps:
i. mixing of tetraethyl orthosilicate (TEOS) precursor which is used as silicon (Si) source, ethanol as solvent, deionized water for hydrolysis and 1 M hydrochloric acid (HCI) at 60-70°C for 2 hours and obtaining SiO₂ sol,
ii. adding aluminum isopropoxide (AIP) precursor used as an aluminum (Al) source into deionized water at 80-90°C and mixing for 1-1.5 hours,
iii. adding 65% nitric acid (HNO₃) to the resulting mixture and mixing for 1-1.5 hours and obtaining Al₂O₃ sol,
iv. adding the SiO₂ sol prepared in the process step (i) into the Al₂O₃ sol and applying the mixing process for 20-30 minutes,
v. adding an aqueous solution of copper nitrate ([Cu(NO₃)₂.3H₂O]) to the resulting mixture and applying the mixing process at 75-95°C for 1-1.5 hours,
vi. slow evaporation of the resulting mixture at a temperature of 50-70°C and gelling the mixture for 3-6 hours,
vii. drying the obtained gel in static air or air flow for 1-24 hours at 90-150°C,
viii. reducing the dried catalyst to 100 mesh - 270 mesh size by grinding and then calcining
   at 300-700°C for 3-9 hours at different temperatures and times.

### REFERENCES

**[1]** DME Fuel Basics. Oberon Fuels. (2021, June 9). Retrieved August 23, 2022, from https://oberonfuels.com/about-dme/dme-basics/
**[2]** Putrasari , Y., & Lim, O. (2021, December 17). Dimethyl Ether as the Next Generation Fuel to Control Nitrogen Oxides and Particulate Matter Emissions from Internal Combustion Engines: A Review. Retrieved from https://pubs.acs.org/doi/10.1021/acsomega.1c03885.
***[3]*** Syngas CO+H2 - air liquide USA. (n.d.). Retrieved August 23, 2022, from https://industry.airliquide.us/sites/activity_us/files/2018/10/16/syngas.pdf
**[4]** ilker, M. A. (2019). Development Of Bifunctional Catalyst For The Single-Step Synthesis Of Dimethyl Ether (thesis).
**[5]** Karbon Dioksit içeriǧi Zengin Sentez Gazιndan iki Fonksiyonlu Katalizor Sistemleriyle Dimetil Eter-Metanol Üretimi. (2018, April). Retrieved August 23, 2022, from https://open.metu.edu.tr/bitstream/handle/11511/49943/TWpBMU5qazU.pdf

## Claims

1. A synthesis method of a bifunctional catalyst for use in one-step synthesis of dimethyl-ether (DME) from CO₂ and H₂ without forming byproduct of CH₄ **characterized by** comprising following steps,
i. mixing of tetraethyl orthosilicate (TEOS) precursor which is used as silicon (Si) source, ethanol as solvent, deionized water for hydrolysis and 1 M hydrochloric acid (HCI) at 50-85°C for 1-5 hours and obtaining SiO₂ sol without precipitation,
ii. adding aluminum isopropoxide (AIP) precursor used as an aluminum (Al) source into deionized water at 75-95°C and mixing for 0.5-3 hours,
iii. adding 65% nitric acid (HNO₃) to the resulting mixture and mixing for 0.5-3 hours and obtaining Al₂O₃ sol without precipitation,
iv. adding the SiO₂ sol prepared in the process step (i) into the Al₂O₃ sol and
applying the mixing process for 15-45 minutes,
v. adding an aqueous solution of copper nitrate ([Cu(NO₃)₂.3H₂O]) to the resulting mixture and applying the mixing process at 75-95°C for 0.5-3 hours,
vi. slow evaporation of the resulting mixture at a temperature of 50-70°C and gelling the mixture for 3-6 hours,
vii. drying the obtained gel in static air or air flow for 1-24 hours at 90-150°C,
viii. reducing the dried catalyst to 100 mesh - 270 mesh size by grinding and then calcining at 300-700°C for 3-9 hours at different temperatures and times.

2. The method according to Claim 1, **characterized by** comprising following steps,
i. mixing of tetraethyl orthosilicate (TEOS) precursor which is used as silicon (Si) source, ethanol as solvent, deionized water for hydrolysis and 1 M hydrochloric acid (HCI) at 60-70°C for 2 hours and obtaining SiO₂ sol,
ii. adding aluminum isopropoxide (AIP) precursor used as an aluminum (Al) source into deionized water at 80-90°C and mixing for 1-1.5 hours,
iii. adding 65% nitric acid (HNO₃) to the resulting mixture and mixing for 1-1.5 hours and obtaining Al₂O₃ sol,
iv. adding the SiO₂ sol prepared in the process step (i) into the Al₂O₃ sol and applying the mixing process for 20-30 minutes,
v. adding an aqueous solution of copper nitrate ([Cu(NO₃)₂.3H₂O]) to the resulting mixture and applying the mixing process at 75-95°C for 1-1.5 hours,
vi. Slow evaporation of the resulting mixture at a temperature of 50-70°C and gelling the mixture for 3-6 hours,
vii. drying the obtained gel in static air or air flow for 1-24 hours at 90-150°C,
viii. reducing the dried catalyst to 100 mesh - 270 mesh size by grinding and then calcining at 300-700°C for 3-9 hours at different temperatures and times.

3. A bifunctional catalyst synthesized by the method according to Claim 1 or 2.

4. A bifunctional catalyst for use in one-step synthesis of dimethyl-ether (DME) using CO₂ and H₂ without forming unwanted byproduct of CH₄ **characterized by** comprising 10%-90% Al₂O₃, 10%-90% SiO₂ and 10%-90% CuO by weight.

5. Use of a bifunctional catalyst according to Claim 4 in one-step synthesis of dimethyl-ether (DME) using CO₂ and H₂ without forming unwanted CH₄ byproduct.
